# EUROPÄISCHE PATENTSCHRIFT

(11) **EP 0 848 103 B1**
(45) Veröffentlichungstag und Bekanntmachung des Hinweises auf die Patenterteilung: **26.02.2003**
(21) Anmeldenummer: 97121128.9
(22) Anmeldetag: 02.12.1997
(51) Int. Cl.: D06M 13/224, D06M 13/463, D06M 13/144, C11D 1/835, C11D 3/00

(54) **Mittel für die Avivage von Textil- und Keratinfasern**
Treatment agent for textile and keratin fibers
Agent d'aviage de fibres textiles et kératiniques

(30) Priorität: 11.12.1996 DE 19651447
(43) Veröffentlichungstag der Anmeldung: 17.06.1998
(73) Patentinhaber: Cognis Deutschland GmbH & Co. KG, 40589 Düsseldorf (DE)
(72) Erfinder: Pi Subirana, Rafael, Dr., 08400 Granollers (ES); Bonastre Gilabert, Nuria, Dr., 08210 Barberá del Vallés (ES); Prat Queralt, Ester, Dr., 08238 Alella (ES); Llosas Bigorra, Joaquim, Dr., 08203 Sabadell (ES)

(56) Entgegenhaltungen:
- EP-A- 0 023 333
- EP-A- 0 023 334
- EP-A- 0 075 770
- EP-A- 0 309 052
- WO-A-96/35661
- WO-A-97/23590
- WO-A-97/29171
- DD-A- 300 605
- DE-A- 3 312 328
- DE-A- 4 437 032

## Beschreibung

### Gebiet der Erfindung

Die Erfindung betrifft Avivagemittel mit einem Gehalt an ausgewählten Hydroxycarbonsäureestern, Esterquats und gegebenenfalls Fettalkoholen sowie die Verwendung der Hydroxycarbonsäureester zur Herstellung von Avivagemitteln.

### Stand der Technik

Nach dem Waschen weisen textile Flächengebilde, also sowohl Garne und Stoffe als auch die daraus hergestellten Textilien, in der Regel eine gewisse Härte auf, die vom Endverbraucher nicht gewünscht wird. Dieser unangenehme Griff kann mechanisch verbessert werden, beispielsweise indem man die Wäsche nach dem Waschen in einem Trockner nachbehandelt, üblicherweise gibt man aber während des Waschvorgangs der Flotte Weichspülmittel hinzu, bei denen es sich in der Regel um kationische Tenside handelt. Die Kationtenside ziehen auf den Fasern auf, erniedrigen die elektrostatische Aufladung zwischen ihnen und führen in der Summe zu einem verbesserten Weichgriff. Was für diese Form der Wäschenachbehandlung gilt, findet ihre Entsprechung bei der Vorbehandlung und Ausrüstung der Garne, Gewebe und Stoffe.

Die gleiche Problemstellung, die aus der Textiltechnik bekannt ist, findet man indes bei einer ganz anderen Art von Fasern: dem menschlichen Haar. Auch Keratinfasem fühlen sich nach dem Shampoonieren häufig hart und stumpf an und lassen sich wegen der elektrostatischen Aufladung schwer entwirren und kämmen. Die gleichen kationischen Tenside, die in der Textilveredlung Anwendung finden, können auch in diesem gänzlich unterschiedlichen Einsatzbereich gelegentlich als Problemlöser dienen.

Nichtsdestotrotz gibt es im Markt ein ständiges Bedürfnis nach neuen Avivagemitteln für Textilien und Haaren, wobei ein besonderes Interesse solchen Stoffen sicher ist, die einen Weichgriff vermitteln, ohne dabei kationisch zu sein, speziell, ohne dabei Stickstoffverbindungen zu enthalten. Insgesamt besteht aus ökotoxikologischen Gründen der Wunsch, Avivagemittel zur Verfügung zu stellen, die entweder völlig frei von Stickstoffverbindungen sind oder nur noch einen vergleichsweise geringen Anteil dieser Stoffe enthalten, sofern die Performance der Verbindungen den Kationtensiden des Stands der Technik wenigstens entspricht. In diesem Zusammenhang sei beispielsweise auf die US-Patentschrift **US 5,290,459** (Colgate) sowie die Deutsche Patentanmeldung **DE-A1 4242689** (Henkel) verwiesen, in denen Pentaerythritester bzw. sulfatierte Partialglyceride als stickstofffreie Avivagemittel vorgeschlagen werden. Diese Stoffe erweisen sich in der Praxis jedoch als weniger effektiv als beispielsweise kationische Tenside vom Esterquat-Typ.

Demzufolge hat die Aufgabe der Erfindung darin bestanden, neue Avivagemittel für Textil- und Keratinfasern zur Verfügung zu stellen, die das oben geschilderte komplexe Anforderungsprofil erfüllen.

### Beschreibung der Erfindung

Gegenstand der Erfindung sind Mittel für die Avivage von Textil- und Keratinfasern, enthaltend
(a) Ester von mehrwertigen Hydroxycarbonsäuren mit Fettalkoholen und
(b) Esterquats sowie gegebenenfalls
(c) Fettalkohole.

Überraschenderweise wurde gefunden, daß Ester auf Basis von mehrwertigen Hydroxycarbonsäuren und Fettalkoholen, insbesondere Citronensäurefettsäureester, über ausgezeichnete avivierende Eigenschaften verfügen, die elektrostatische Aufladung zwischen den Fasern herabsetzen und ökotoxikologisch unbedenklich sind. Werden die Ester alleine eingesetzt, werden stickstoftfreie Avivagemittel für Textilien und Haare erhalten. In einer bevorzugten Ausführungsform der Erfindung lassen sich die Ester jedoch mit geringen Mengen an kationischen Tensiden vom Esterquat-Typ kombinieren, wobei stickstoffarme Avivagemittel mit synergistisch verbessertem Leistungsspektrum erhalten werden.

### Hydroxycarbonsäureester

Hydroxycarbonsäureester, die die Komponente (a) bilden, stellen bekannte Stoffe dar, die man üblicherweise durch Veresterung der Säuren mit Fettalkoholen mit 6 bis 22 Kohlenstoffatomen in Gegenwart von sauren Katalysatoren wie beispielsweise Hypophosphoriger Säure, Natriumhypophosphit oder Methansulfonsäure herstellt. Typische Beispiele für geeignete mehrwertige Hydroxycarbonsäuren sind Weinsäure, Äpfelsäure und insbesondere Citronensäure. Als Fettalkohole kommen für die Veresterung in Frage: Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern-, Cetylstearyl- oder Talgfettalkohol. Das molare Einsatzverhältnis von Hydroxycarbonsäure zu Fettalkohol kann 1 : 0,5 bis 1 : 1,3, vorzugsweise 1:1 bis 1:1,25 - bezogen auf die jeweilige Anzahl von Carbonsäurefunktionen - betragen. Im Verlauf der Reaktion kann es neben der Bildung von Estem aus Hydroxycarbonsäure und Alkohol auch zur Bildung von inneren Estem der Hydroxycarbonsäuren kommen, die ihrerseits noch freie Carboxylgruppen enthalten und mit Alkoholen weiterreagieren können.

### Esterquats

Unter Esterquats, die die Komponente (b) bilden, sind quatemierte Fettsäuretriethanolaminestersalze zu verstehen. Es handelt sich dabei um bekannte Stoffe, die man nach den einschlägigen Methoden der präparativen organischen Chemie erhalten kann. In diesem Zusammenhang sei auf die Intemationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 4308794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am.Oil.Chem.Soc., 71, 97 (1994)** sowie I.Shapiro in **Cosm.Toil. 109, 77 (1994)** erschienen.

Die quaternierten Fettsäuretriethanolaminestersalze folgen der Formel **(I),** 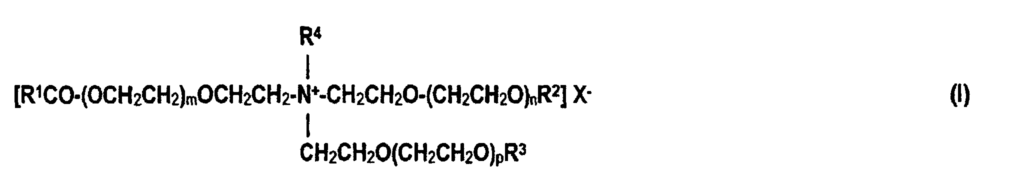 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2:1 bis 292:1, vorzugsweise 1,5:1 bis 1,9:1 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (lodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quatemierten Fettsäuretriethanolaminestersalzen kommen als Esterquats ferner auch quaternierte Estersalze von Fettsäuren mit Diethanolalkylaminen der Formel **(II)** in Betracht, 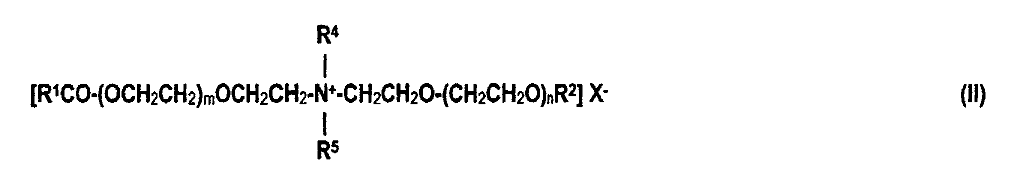 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 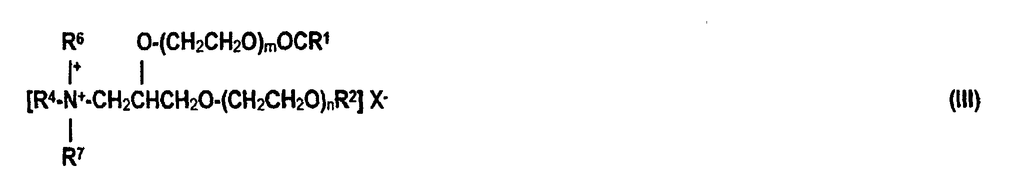 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können. Vorzugsweise enthalten die erfindungsgemäßen Mittel als Komponente (b) methylquaternierte Triethanolaminfettsäureester.

### Fettalkohole

Typische Beispiele für Fettalkohole, die die Komponente (c) bilden, sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 6 bis 22, insbesondere 12 bis 18 Kohlenstoffatomen wie beispielsweise Kokos-, Palm-, Palmkern-, Cetylstearyl- oder Talgfettalkohol.

### Gewerbliche Anwendbarkeit

Die erfindungsgemäßen Mittel verleihen Textilien und Haaren einen angenehmen Weichgriff und vermindern die elektrostatische Aufladung zwischen den Fasern. Sie eignen sich daher zur Herstellung von Mitteln zur Faseravivage, insbesondere Wäscheweichspülmitteln sowie für haarkosmetische Zubereitungen, wie z.B. Shampoos, Conditionern und dergleichen. In einer bevorzugten Ausführungsform der Erfindung enthalten die Mittel
(a) 10 bis 90, vorzugsweise 60 bis 80 Gew.-% Ester von mehrwertigen Hydroxycarbonsäuren mit Fettalkoholen,
(b) 10 bis 50, vorzugsweise 10 bis 30 Gew.-% Esterquats und
(c) 1 bis 15, vorzugsweise 5 bis 10 Gew.-% Fettalkohole,
mit der Maßgabe, daß sich die Komponenten zu 100 Gew.-% addieren.

Ein weiterer Gegenstand der Erfindung betrifft die Verwendung von Estern von mehrwertigen Hydroxycarbonsäuren mit Fettalkoholen zur Herstellung von Mitteln für die Avivage von Textil- und Keratinfasern.

Die erfindungsgemäßen Zubereitungen können in untergeordneten Mengen weitere, mit den anderen Inhaltsstoffen kompatible Tenside enthalten. Typische Beispiele sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkyloligoglucoside, Fettsäureglucamide, Alkylamidobetaine und/oder vorzugsweise pflanzliche Proteinfettsäurekondensate.

Insbesondere dann, wenn die Zubereitungen Mittel zu Haarbehandlung darstellen, können ferner als weitere Hilfs- und Zusatzstoffe Ölkörper, Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe enthalten sein.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether, Siliconöle undloder aliphatische bzw. naphthenische Kohlenwasserstoffe in Betracht.

Als **Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 1943689, DE-OS 2036472** und **DE-A1 3001064** sowie **EP-A 0077167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als Konsistenzgeber kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), quatemierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als Stabilisatoren können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

### Herstellbeispiel 1:

In einem 1-l-Dreihalskolben mit Rührer und Destillationsaufsatz wurden 192 g (1 Mol) Citronensäure und 640 g (2,5 Mol) Cetylstearylalkohol vorgelegt und mit 2,1 g Natriumhypophosphit (0,25 Gew.-% bezogen auf die Einsatzstoffe) versetzt. Die Mischung wurde auf 185°C erhitzt und das Kondensationswasser bei einem Vakuum von etwa 40 mbar kontinuierlich entfernt. Nach einer Reaktionszeit von ca. 240 min wurde der Citronensäurecetylstearylester in Form einer weißen, schuppbaren Masse erhalten. Die Restsäurezahl des Produktes betrug 24,3 mg KOH/g. Das Produkt ließ sich bei 35°C zu einer stabilen, dünnflüssigen milchig-weißen Dispersion mit einem Aktivsubstanzgehalt von 5 Gew.-% verarbeiten.

### Herstellbeispiel 2:

Beispiel 1 wurde unter Einsatz von 192 g Citronensäure und 768 g (3 Mol) Cetylstearylalkohol wiederholt. Der resultierende Citronensäureester wies eine Rest-Säurezahl von 8,5 auf. Das Produkt ließ sich bei 35°C zu einer stabilen, dünnflüssigen milchig-weißen Dispersion mit einem Aktivsubstanzgehalt von 5 Gew.-% verarbeiten.

### Anwendungstechnische Beispiele 3 bis 7 sowie Vergleichsbeispiele V1 bis V3

Baumwollgewebe wurde mit einem handelsüblichen Universalwaschmittel gewaschen, wobei man in den letzten Waschgang Weichspülmittel der Zusammensetzungen gemäß Tabelle 1 hinzugab. Anschließend wurde der Weichgriff der Gewebe von einem Panel aus 6 geschulten Personen beurteilt. Die Beispiele 3 bis 7 sind erfindungsgemäß, die Beispiele V1 bis V3 dienen zum Vergleich. Bezüglich des Weichgriffes bedeutet (1) sehr weich und 5 (hart).

**Tabelle 1**

| **Weichgriff verschiedener Avivagemittel** | | | | | | | | |
|---|---|---|---|---|---|---|---|---|
| **Zusammensetzung** | **3** | **4** | **5** | **6** | **7** | **V1** | **V2** | **V3** |
| Citronensäuretricetylstearylester (Bsp.1) | 100 | - | - | - | - | - | - | - |
| Citronensäuretricetylstearylester (Bsp.2) | - | 100 | 70 | 70 | 67 | - | - | - |
| Pentaerythrittetracetylstearylester | - | - | - | - | - | 100 | 70 | - |
| Methylquarternierter Triethanolaminbistalgfettsäureester-Methylsulfatsalz | - | - | 30 | - | - | - | - | - |
| Methylquarternierter Triethanolaminmonotalgfettsäureester-Methylsulfatsalz | - | - | - | 30 | 25 | - | 30 | 100 |
| Cetylsterarylalkohol | - | - | - | - | 8 | - | - | - |
| ***Weichgriff*** | 2,0 | 2,0 | 1,5 | 1,5 | 1,0 | 3,0 | 2,5 | 2,5 |

## Patentansprüche

1. Mittel für die Avivage von Textil- und Keratinfasern, enthaltend
(a) Ester von mehrwertigen Hydroxycarbonsäuren mit Fettalkoholen und
(b) Esterquats sowie gegebenenfalls
(c) Fettalkohole.

2. Mittel nach Anspruch 1, **dadurch gekennzeichnet, daß** sie als Komponente (a) Ester der Citronensäure enthalten.

3. Mittel nach den Ansprüchen 1 und 2, **dadurch gekennzeichnet, daß** sie als Komponente (a) Ester von Fettalkoholen mit 12 bis 18 Kohlenstoffatomen enthalten.

4. Mittel nach den Ansprüchen 1 bis 3, **dadurch gekennzeichnet, daß** sie als Komponente (b) methylquaternierte Triethanolaminfettsäureester enthalten.

5. Mittel nach den Ansprüchen 1 bis 4, **dadurch gekennzeichnet, daß** sie als Komponente (c) Fettalkohole mit 12 bis 18 Kohlenstoffatomen enthalten.

6. Mittel nach den Ansprüchen 1 bis 5, **dadurch gekennzeichnet, daß** sie
(a) 10 bis 90 Gew.-% Ester von mehrwertigen Hydroxycarbonsäuren mit Fettalkoholen,
(b) 10 bis 50 Gew.-% Esterquats und
(c) 1 bis 15 Gew.-% Fettalkoholen
enthalten, mit der Maßgabe, daß sich die Komponenten zu 100 Gew.-% addieren.

7. Verwendung von Estern von mehrwertigen Hydroxycarbonsäuren mit Fettalkoholen zur Herstellung von Mitteln für die Avivage von Textil- und Keratinfasern.

## Claims

1. Preparations for conditioning textile and keratin fibres containing
(a) esters of polybasic hydroxycarboxylic acids with fatty alcohols and
(b) esterquats and optionally
(c) fatty alcohols.

2. Preparations as claimed in claim 1, **characterized in that** they contain esters of citric acid as component (a).

3. Preparations as claimed in claims 1 and 2, **characterized in that** they contain esters of C₁₂₋₁₈ fatty alcohols as component (a).

4. Preparations as claimed in claims 1 to 3, **characterized in that** they contain methyl-quaternized triethanolamine fatty acid esters as component (b).

5. Preparations as claimed in claims 1 to 4, **characterized in that** they contain C₁₂₋₁₈ fatty alcohols as component (c).

6. Preparations as claimed in claims 1 to 5, **characterized in that** they contain
(a) 10 to 90% by weight of esters of polybasic hydroxycarboxylic acids with fatty alcohols,
(b) 10 to 50% by weight of esterquats and
(c) 1 to 15% by weight of fatty alcohols,
with the proviso that the component add up to 100% by weight.

7. The use of esters of polybasic hydroxycarboxylic acids with fatty alcohols for the production of preparations for conditioning textile and keratin fibres.

## Revendications

1. Agent d'avivage (ou d'ensimage) de fibres textiles et kératiniques, contenant
(a) des esters d'acides hydroxycarboxyliques polyvalents avec des alcools gras et
(b) des esterquats ainsi que le cas échéant
(c) des alcools gras.

2. Agents selon la revendication 1,
**caractérisés en ce qu'**
ils contiennent comme composant (a) des esters de l'acide citrique.

3. Agents selon les revendications 1 et 2,
**caractérisés en ce qu'**
ils contiennent comme composant (a) des esters d'alcools gras comportant de 12 à 18 atomes de carbone.

4. Agents selon les revendications 1 à 3,
**caractérisés en ce qu'**
ils contiennent comme composant (b) des esters d'acides gras de triéthanolamine méthyl-quaternisés.

5. Agents selon les revendications 1 à 4,
**caractérisés en ce qu'**
ils contiennent comme composant (c) dès alcools gras comportant de 12 à 18 atomes de carbone.

6. Agents selon les revendications 1 à 5,
**caractérisés en ce qu'**
ils contiennent
(a) de 10 à 90 % en poids d'esters d'acides hydroxycarboxyliques polyvalents avec des alcools gras,
(b) de 10 à 50 % en poids d'esterquats et
(c) de 1 à 15 % en poids d'alcools gras,
sous réserve que la somme des composants est de 100 % en poids.

7. Utilisation d'esters d'acides hydroxycarboxyliques polyvalents avec des alcools gras pour la production d'agents pour l'avivage (ou ensimage) des fibres textiles et kératiniques.
